# EUROPEAN PATENT SPECIFICATION

(11) **EP 4 440 462 B1**
(45) Date of publication and mention of the grant of the patent: **29.04.2026**
(21) Application number: 22823495.1
(22) Date of filing: 30.11.2022
(51) Int. Cl.: A61B 18/22

(54) **ABLATING APPARATUS**
ABLATIONSVORRICHTUNG
APPAREIL D'ABLATION

(30) Priority: 01.12.2021 IT 202100030410; 28.04.2022 US 202263335786 P
(43) Date of publication of application: 09.10.2024
(73) Proprietor: Alleima Karlsruhe GmbH, 76131 Karlsruhe (DE)
(72) Inventor: GIULIANI, Laura, Varese (IT)
(74) Representative: Alleima
(86) International application number: PCT/EP2022/083888
(87) International publication number: WO 2023/099582

(56) References cited:
- US-A1- 2017 027 640
- US-A1- 2019 104 933
- US-A1- 2019 282 299

## Description

The invention relates to a medical apparatus for supporting and centering a treatment device configured to treat the internal walls of a longitudinal cavity, in particular a human cavity. In addition, the invention relates to an ablating and resurfacing system comprising said medical apparatus and a treatment device for the ablation and the resurfacing of the superficial mucosae of a human cavity, in particular the gastro apparatus, more particularly the duodenum. Also, the invention relates to an endoscopic device comprising the medical apparatus or the ablating and resurfacing system.

Type II diabetes is the most prevalent metabolic disease worldwide with a huge impact on the quality life of patient and extremely high cost for the society and the healthcare systems. When adopting a healthier lifestyle does not suffice, Type II diabetes is commonly treated through medications, drugs and surgery.

Obesity is often strictly associated with type II diabetes. It is well known from bariatric surgery clinical studies, that surgeries on the gastrointestinal (Gl) tract can contribute to the improvement of insulin resistance and type II diabetes disease extent. Examples of this surgery include gastric and duodenal bypass that present high risk for patients, high cost for caregivers and are not a viable options for non-obese patients or patients in an early stage of the disease.

Several solutions were developed overtime to mimic the effect of bariatric surgery, including:
- physical barriers that prevent the contact between food and duodenal mucosae;
- duodenal mucosae resurfacing (MDR) device based on a balloon inflated with hot liquid; and
- laser devices to target the neural activity in the duodenum.

However, all of them have limitations and side effects. For example, the use of physical barriers can cause nausea, pain, mucosal tear, bleeding, migration and obstruction, with the necessity of device removal overtime. This being therefore not a possible permanent solution. The use of MDR devices can lead to uncontrolled depth of thermal damage to the surface. Also, there is a lack of real time visibility of the portion of tissue actually treated by the surgeon. On the other hand, the employment of laser devices is targeted to the nerve system rather than the mucosae. Also in this case, there is a lack of real time visibility of the portion of tissue actually treated by the surgeon. Furthermore, it is not always possible to ensure an homogeneous delivery of power energy on the tissue to be treated, thereby leading to undesired effects. In this context, reference is made to document US2019/0282299 A1.

WO 2018/033910 describes for example an apparatus for providing feedback to nerve ablation in the wall of the gastrointestinal tract using laser ablation. In particular, the apparatus includes an expandable balloon used for ensuring that the catheter inserted into the duodenum is positioned in the center at the region designed for treatment. Also, the apparatus comprises a laser emitting element to transmit two laser beams for the ablation of the tissue and for detecting the modifications resulting from the impingement of the first laser beam. In order to precisely direct the laser beam, the apparatus finally comprises a deflective optical element coupled with the laser emitting element. Although effective, this apparatus is made of several different components that make it prone to possible malfunction and wear. Also, the laser requires a dedicated optics in order to be correctly directed to the tissue.

Examples of the present disclosure seek to address or at least alleviate the above problems.

The invention is defined in the appended set of claims.

According to a first aspect of the invention there is provided a medical apparatus for supporting and centering a treatment device treating the internal walls of a longitudinal cavity, in particular a human cavity, the apparatus comprising:
a longitudinal sheath insertable in a working channel of an endoscopic device; and
a positioning element comprising a first end connectable to the longitudinal sheath and having a central channel as well as a first guiding through-hole, a second end configured to be movable from the first end and from the longitudinal sheath and having a second guiding through-hole, and a deformable element fixable to at least the second end;
wherein in a first configuration, or retracted configuration, the second end of the positioning element is at a first distance from the longitudinal sheath, the first guiding through-hole and the second guiding through-hole being aligned for receiving the treatment device and the deformable element is completely compressed in the direction of the longitudinal sheath, and in a second configuration, or expanded configuration, the second end of the positioning element is spaced apart from the longitudinal sheath at a second distance, wherein the first distance is different from the second distance and the deformable element is radially expanded, thereby touching, at least partially, the internal walls of the cavity so that the first guiding through-hole and the second guiding through-hole are moved away from the internal walls of the cavity and the treatment device, when inserted into the first guiding through-hole and the second guiding through-hole, is moved towards a central region of the cavity and is maintained in said position during the treatment.

In a second aspect of the invention there is provided an ablating and resurfacing system for the ablation and the resurfacing of the superficial mucosae of a human cavity, in particular the gastro apparatus, more particularly the duodenum, the system comprising:
a medical apparatus according to according to the first aspect of the invention, and
a treatment device treating the internal walls of the human cavity,
wherein in the first configuration of the apparatus, the treatment device is extended through the first guiding through-hole and the second guiding through-hole and in the second configuration of the apparatus, the second end of the positioning element is moved along the treatment device until the deformable element is expanded and the treatment device is moved away from the internal walls of the cavity, in particular towards a central region of the cavity.

In a third aspect of the invention there is provided an endoscopic device having a working channel and comprising an apparatus according to the first aspect of the invention or a system according to the second aspect of the invention, the apparatus or the system being insertable in the working channel of the endoscopic device.

The invention may be used in a method for treating the internal walls of a longitudinal cavity, in particular a human cavity, by means of the apparatus of the first aspect, or of the system according to the second aspect or the endoscopic device of the third aspect.

Other aspects and features are defined in the appended claims.

Examples of the disclosure may make it possible to obtain a reliable apparatus usable for ablation and resurfacing of a tissue in the gastrointestinal region that ensures a correct treatment of the region and a real time visibility of the portion of tissue actually treated by the surgeon.

Examples of the disclosure will now be described by way of example only with reference to the accompanying drawings, in which like references refer to like parts, and in which:
- Figures 1A-D: show the medical apparatus without the treatment device in the first and second configuration and the medial apparatus with the treatment device in the second configuration according to a offset arrangement (1A-C) and according to a coaxial arrangement (1D).
- Figures 2A-C: show the medical apparatus used in an endoscopic device according to an example, wherein the positioning element is in the first and second configuration of the offset arrangement (2A-B), and wherein the positioning element is in the second configuration of the coaxial arrangement (2C).
- Figures 3A-D: show the medical apparatus receiving a treatment device in different steps according to an example of the offset arrangement.
- Figures 4A-B: show the medical device according to another example with the positioning element in the second configuration of the offset arrangement.
- Figures 5A-G: show the functioning of the medical device inserted in a longitudinal cavity of a human body.
- Figures 6A-C: show detail of connecting ports according to different examples.
- Figures 7A-B: show the passage from the first configuration to the second configuration of the positioning element according to an example of the offset arrangement.
- Figures 8A-C: show the passage from the first configuration to the second configuration of the positioning element according to another example of the offset arrangement.
- Figures 9A-C: show the passage from the first configuration to the second configuration of the positioning element according to an example of the coaxial arrangement.
- Figures 10A-D: show the passage from the first configuration to the second configuration of the positioning element according to an additional example of the coaxial arrangement.
- Figure 11: shows a perspective view of the medical apparatus according to an example of the coaxial arrangement
- Figures 12A-C: show different configurations of deformable element according to an example of the coaxial arrangement.

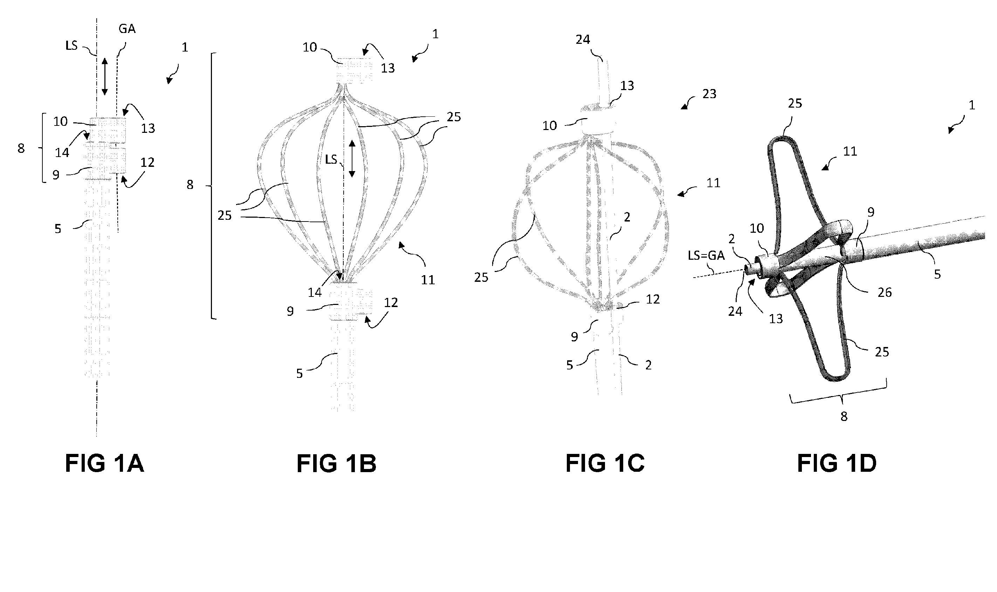

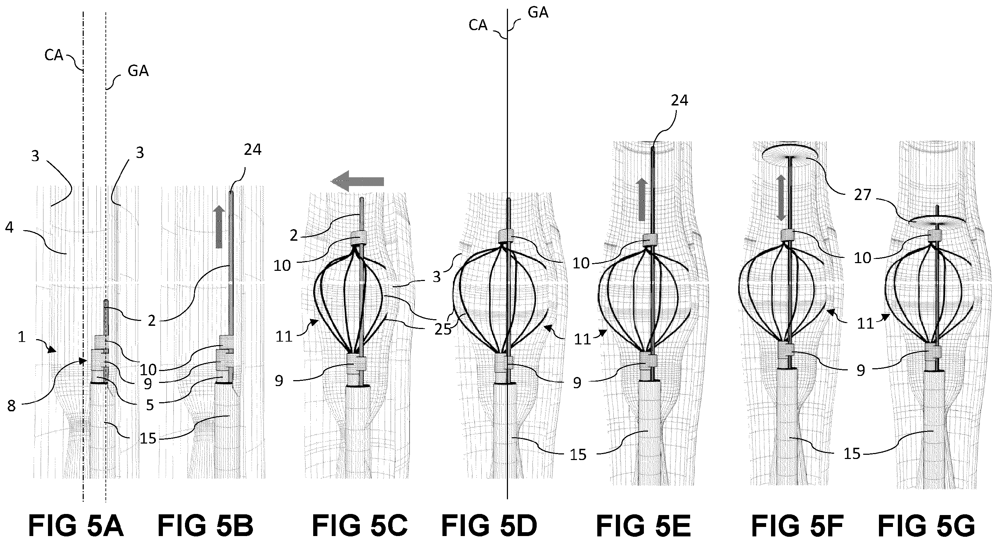

With reference to figures 1A to 1D, the apparatus 1 is suitable for supporting and centering a treatment device 2 treating the internal walls 3 of a human cavity. With treatment device 2 is intended any type of device commonly used for example in the endoscopic procedures that is able to treat the tissue of a human cavity. For example, the treatment can be carried out using light emitting means, thermoelectric means, cutting means, magnetic means, etc. The application of the medical apparatus 1 in the human cavity will be better illustrated in other figures, for example figures 5A-5G.

The apparatus 1 can be set up according to at least two different arrangements, an offset arrangement and a coaxial arrangement, as will be explained in detail below. It is noted that the main difference between the offset arrangement and the coaxial arrangement basically relates to the different position of the treatment device 2 with respect to a central axis of the apparatus 1. Apart this main structural difference, the majority of characteristics and corresponding applications defining the apparatus 1 according to the offset arrangement can also apply to the apparatus 1 according to the coaxial arrangement.

In the offset arrangement (Fig. 1A-1C), the medical apparatus 1 comprises a longitudinal sheath 5 and a positioning element 8 connected to one end of the longitudinal sheath 5. The positioning element 8 comprises a first end 9 that can be connected to the longitudinal sheath 5 and a second end 10. The first end 9 is provided with a central channel 14 and a first guiding through hole 12 located next to, and spaced from, the central channel 14, whereas the second end 10 is provided with a second through-hole 13. According to this example, the central channel 14 is spaced from the first guiding through-hole 12 and the second guiding through-hole 13 is not centered on the second end 10. Therefore, an offset is present between the central channel 14 and the guiding through holes 12, 13.

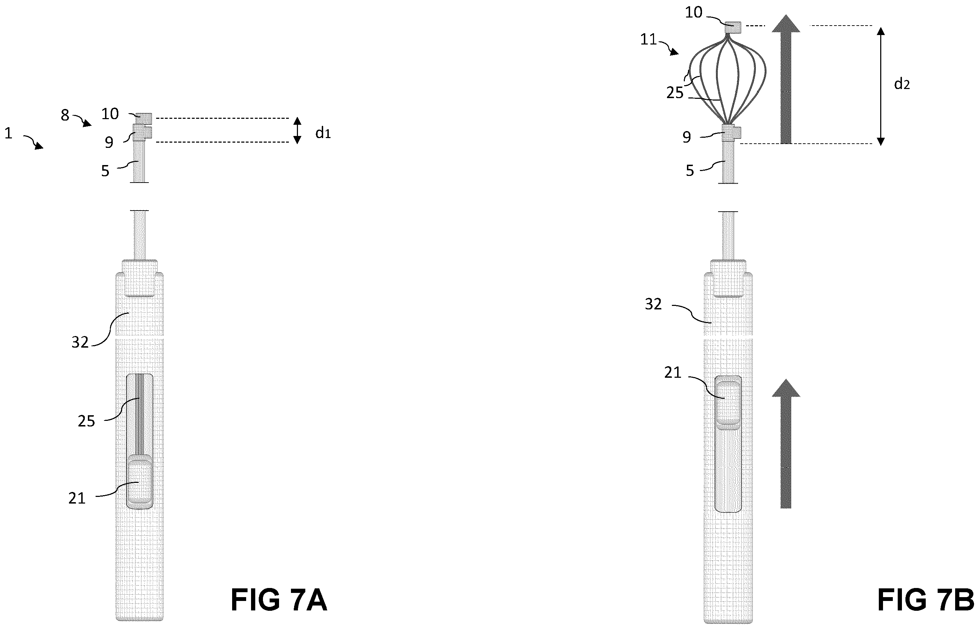

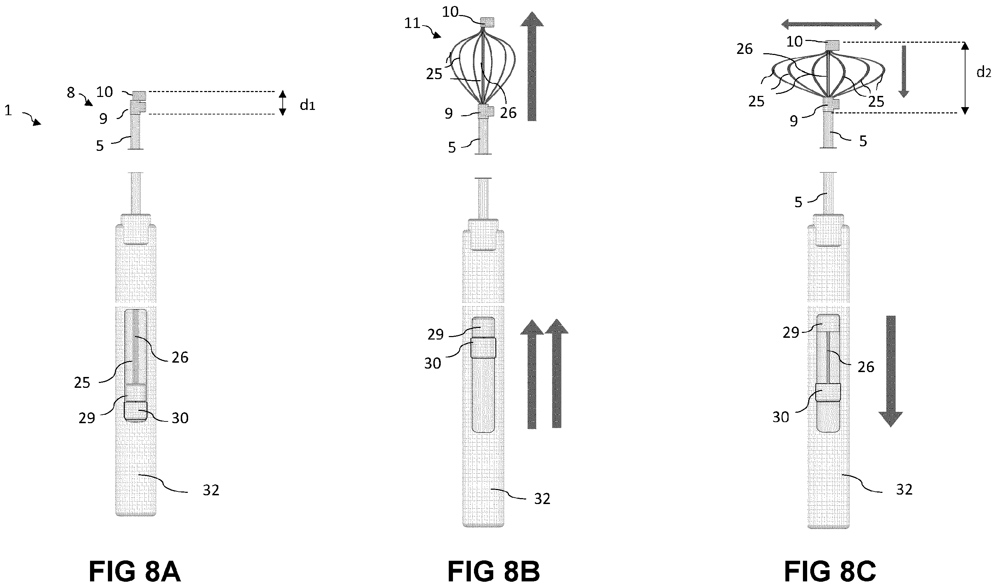

The second end 10 can be moved from the longitudinal sheath 5 and then from the first end 9 so that the positioning element 8 can assume a first configuration or retracted configuration, as shown in figure 1A, and a second configuration or expanded configuration, as shown in figure 1B. The movement occurs along the longitudinal axis LS of the longitudinal sheath 5, as shown with the double arrow in the figure. The deformable element 11 of the positioning element 8 is configured to slide through the central channel 14 of the first end 9 so that in the first configuration, the second end 10 of the positioning element 8 is in contact with the first end 9 of the positioning element 8. As will be shown in more detail with reference to figures 7A-B and 8A-C, the distance between second end 10 and the longitudinal sheath 5 varies passing form the first configuration to the second configuration. In particular, in the first configuration this distance (first distance d₁) is smaller than in the second configuration (second distance d₂) and the deformable element 11 is compressed inside the longitudinal sheath 5. The medical apparatus 1 has therefore an elongated shape like a rod. In this way, it is possible to easily insert the medical device 1 inside a human cavity. As shown in figure 1A, in this configuration, the first guiding through-hole 12 and the second guiding through-hole 13 are aligned. The vertical dashed line in the figure shows the presence of a single passage through the two guiding through-holes 12, 13. This is useful for the insertion of a treatment device 2. Advantageously, the treatment device 2 has an elongated shape suitable for passing through this passage. This vertical dashed axis can be denoted as a guiding axis GA and is arranged parallel to the longitudinal axis LS of the longitudinal sheath 5.

In the second configuration, the first end 9 of the positioning element 8 is spaced apart from the second end 10 of the positioning element 8. In particular, the second end 10 is moved forward along the direction of the longitudinal sheath 5, i.e., along the longitudinal axis LS. As shown in figure 1B, the positioning element 8 furthermore comprises a deformable element 11 fixable to the second end 10. As mentioned above, the deformable element 11 is configured to slide through the central channel 14 of the first end 9. It is noted that in the first configuration the deformable element 11 is completely compressed inside at least the longitudinal sheath 5, and in a second configuration the deformable element 11 is radially expanded. In other words, moving the second end 10 away from the first end 9 causes the deformable element 11 to be extracted from the longitudinal sheath 5 through the central channel 14 and to be expanded.

According to an example (e.g. figure 1B), the deformable element 11 comprises a plurality of deformable threads 25. Each thread 25 can extend from the second end 10 to the first end 9. It is noted that the threads 25 are fixed at the second end 10 but are not fixed to the first end 9 of the positioning element 8. As a matter of fact, the threads 25 can be configured to pass through the central channel 14 and can be fixed at an internal portion of the longitudinal sheath 5 (not shown in the figure). In an example, the plurality of deformable threads 25 are made of a material having shape memory and superelasticity, in particular of a metal alloy of nickel and titanium (i.e. Nitinol). Of course, the deformable element 11 can be made of different materials that are suitable for the purpose of the present disclosure.

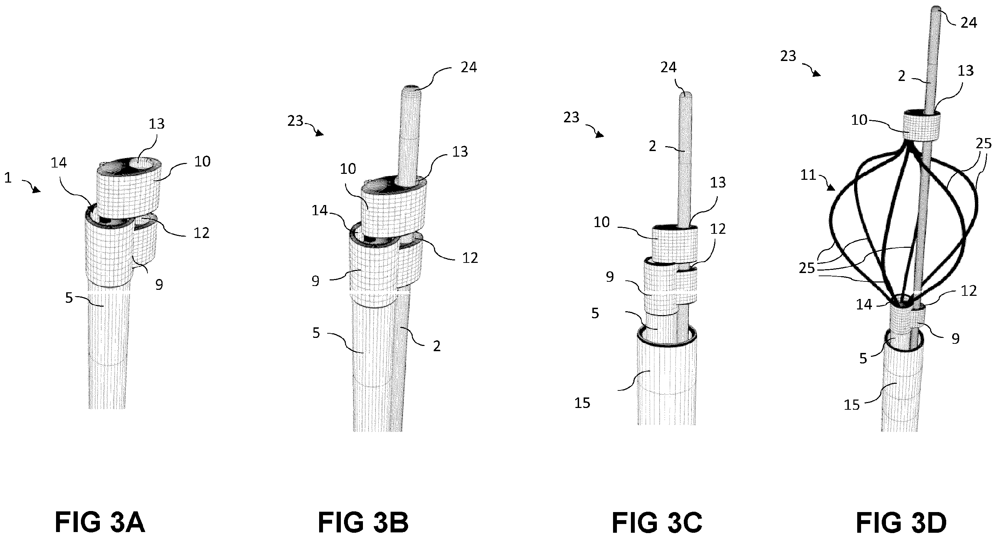

The first and second guiding through-holes 12, 13 are configured for receiving a treatment device 2. Advantageously, the treatment device 2 is inserted in the guiding through-holes 12, 13 when the positioning element 8 is in the first configuration, as will be explained in detail in figures 3A-3D. Figure 1C shows on the other hand, the situation wherein the treatment device 2 is inserted into the two guiding through-holes 12, 13 and the positioning element 8 is in the second expanded configuration. The combination of the medical apparatus 1 with the treatment device 2 can be defined as an ablating and resurfacing system 23. As will be explained in more detail with the following figures, in the second configuration of the apparatus 1, the second end 10 of the positioning element 8 is moved along the treatment device 2 until the deformable element 11 is expanded. In this way, the first and second guiding through-holes 12, 13, and consequently the treatment device 2, are moved away from the internal walls 3 of the human cavity 4, in particular towards a central region of the human cavity 4.

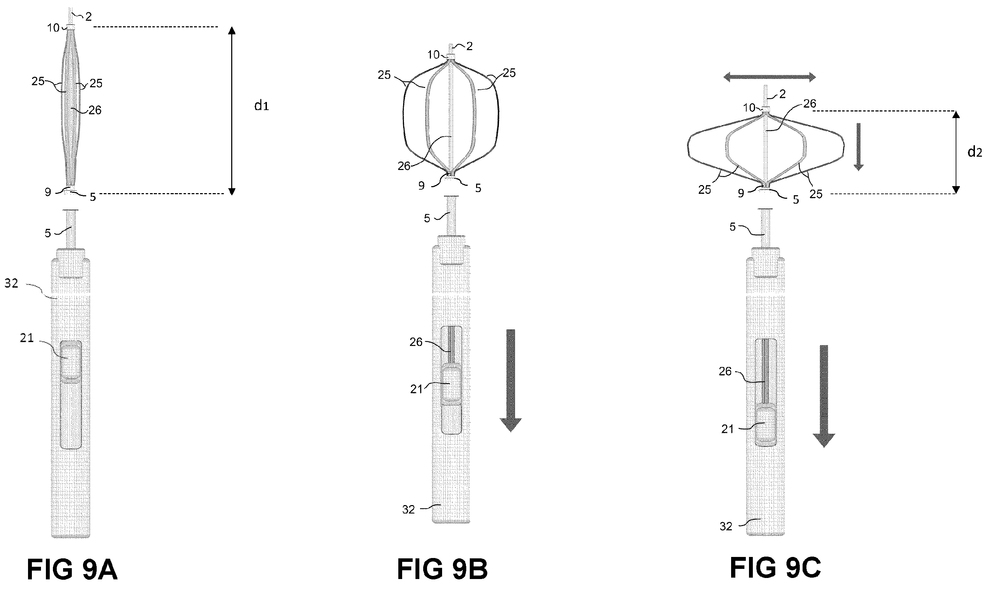

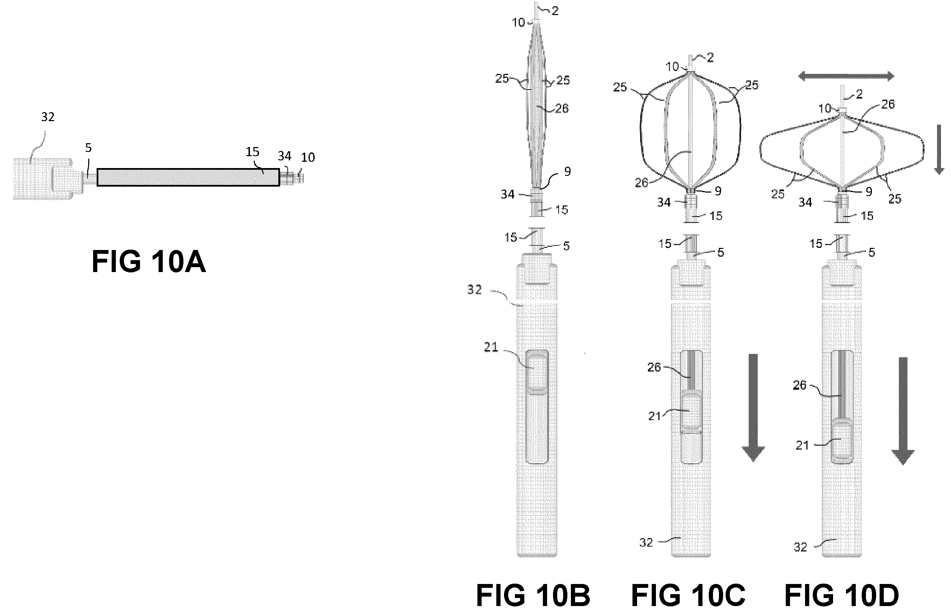

In an alternative example, the first guiding through-hole 12 is coaxial with the central channel 14 and the second guiding through-hole 13 is centered on the second end 10 (coaxial configuration). This is shown for example in figure 1D. In this case, according to the coaxial arrangement, the guiding axis GA coincides with the longitudinal axis LS of the longitudinal sheath 5 and the treatment device 2 can slide along the longitudinal axis LS of the longitudinal sheath 5. It is noted that the deformable element 11 is fixable also to the first end 9 of the positioning element 8 so that that in the first configuration the second end 10 of the positioning element 8 is moved away from the first end 9 of the positioning element 8 so that that the first distance d₁ is greater than the second distance d₂ and the deformable element 11 is compressed outside the longitudinal sheath 5. Figure 1D only shows the deformable element 11 in the second or expanded configuration. The first or retracted configuration for this coaxal arrangement is illustrated in figures 9A-9C and 10A-10D.

It is noted that similarly to offset arrangement, the deformable element 11 in the coaxial arrangement can comprise a plurality of deformable threads 25. Each thread 25 can extend from the second end 10 to the first end 9. It is noted that the threads 25 are fixed to both the first end 9 and to the second end 10 of the positioning element 8. As mentioned above, the plurality of deformable threads 25 can be made of a material having shape memory and superelasticity, in particular of a metal alloy of nickel and titanium. Of course, the deformable element 11 can be made of different materials that are suitable for the purpose of the present disclosure.

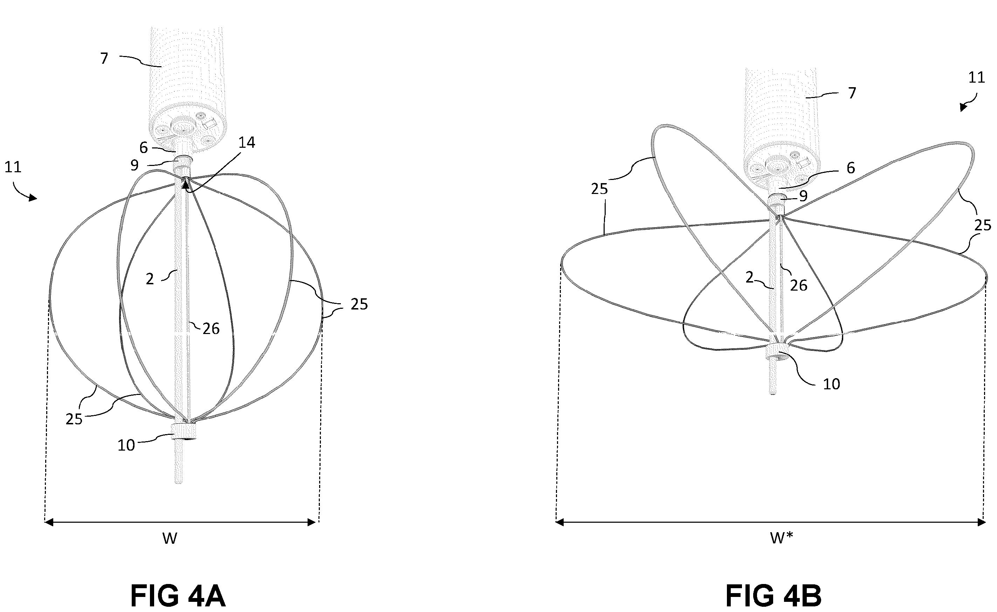

In examples for both the offset and coaxial arrangement, in the second configuration, the deformable element 11 can assume a basket-shaped structure. Of course, the deformable element 11 can be configured in several different ways. For example, the deformable element 11 can comprise one or more components able to vary their shape from a contracted to an extended configuration after the supply of a fluid (gas or liquid). The deformable element 11 can have the shape of a balloon. In case of the presence of a distance varying element 26, the deformable element 11 can be changed from an almost round shape to a more compressed, flattened shape. The presence of a distance varying element 26 will be described for example with reference to figures 4A-4B.

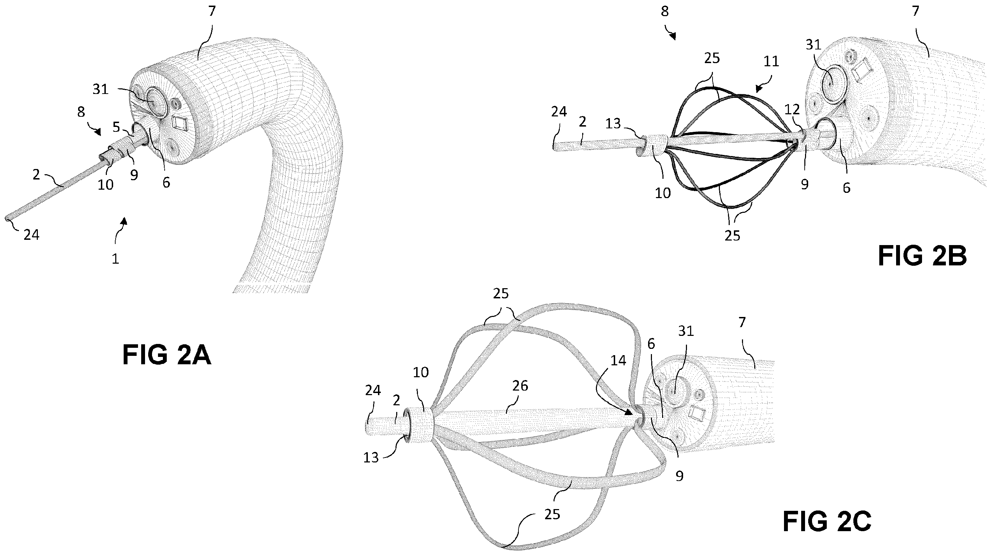

Figures 2A, 2B and 2C illustrate the application of the medical apparatus 1 in an endoscopic device 7. The endoscopic device 7 has at least a working channel 6 into which the medical apparatus 1, or the system 23, is inserted. The device 7 can be an endoscope for gastroscopy, in particular a flexible endoscope. In particular, figures 2A and 2B illustrate the application of the medical apparatus 1 of figures 1A-1C, i.e. in the offset arrangement, whereas figure 2C illustrates the application of the medical apparatus 1 of figure 1D, i.e. in the coaxial arrangement.

In figure 2A the positioning element 8 of the medical apparatus 1 is in the retracted configuration. As a matter of fact, the first end 9 is in contact with the second end 10 of the positioning element 8 and the medical apparatus 1 can be easily moved through the working channel 6. On the other hand, in figure 2B, the positioning element 8 of the medical apparatus 1 is in the expanded configuration. As a matter of fact, the second end 10 is spaced apart from the first end 9 and the deformable threads 25 of the deformable element 11 are expanded forming a basked-shaped structure. The endoscopic device 7 can be inserted in a human cavity (e.g. duodenum) when the positioning element 8 is in the first configuration. When it is found the tract of the human cavity to be treated, the positioning element 8 can be moved to the second configuration so that the deformable thread can touch the internal walls of the cavity and can cause the movement of the treatment device 2 from a region of the cavity close to the internal walls towards the central region of the cavity. It is noted that the deformable element 11 in the extended configuration can also serve to maintain the treatment device 2 in position during the treatment, when required.

The endoscopic device 7 can have all the features typical of a conventional device used in endoscopic surgery. For example, the device 7 can comprise a camera 31 for monitoring the effect of the treatment device 2 on the internal walls 3 of the cavity 4. The monitoring can be carried out on real-time basis.

In one example, the treatment device 2 is a laser system, in particular emitting a radiation having a wavelength comprised between 1900 nm and 2100 nm or between 10200 nm and 10600nm. For example, the treatment device 2 can be a laser optical fiber, in particular a thulium fiber laser. This type of laser serves to ablate the surface of the target tissue conserving the surrounding tissue thanks to the superficial precise action of this specific wavelength. The photo-thermal effects indeed increase the temperature of the water contained on the tissue causing the ablation or denaturation of the tissue without deep propagation of the thermal effect on the surrounding tissues. With laser optical fiber is intended here a laser generator for generating a laser radiation coupled to an optical fiber for conducting the laser radiation through the human cavity, only the optical fiber being inserted into the cavity. Advantageously, the optical fiber can comprise one or more emitting points, usually on the tip, for emitting the laser radiation.

In another example, the treatment device 2 can be an optical fiber laser system comprising a laser generator 28 coupled to an optical fiber structure 33 having a tip portion 24 for a diffusive or radial laser emission.

Figure 2C shows the positioning element 8 in the expanded configuration of an alternative medical apparatus 1 in the coaxial arrangement. In particular, the medical apparatus 1 of figure 2C has the same characteristics of the apparatus 1 of figure 2B with the difference that the treatment device 2 is not offset with respect to the central channel 14 but is coaxial with this channel.

Figures 3A-3D illustrate the medical apparatus 1 and the ablating system 23 according to two examples of the offset arrangement. In a first example (figures 3A-3B) the apparatus 1 and the system 23 correspond to the apparatus and system already described in figures 1A-C. In a second example (figures 3C-3D), the apparatus 1 and the system 23 further comprise an external sheath 15 containing both the longitudinal sheath 5 and the treatment device 2. The external sheath 15 can be inserted in the working channel 6 of the endoscopic device 7. Since the external sheath 15 contains both the medical apparatus 1 and the treatment device 2, the sheath 15 is useful to facilitate the insertion in the working channel 6 of the endoscopic device 7.

Figures 4A and 4B illustrates the medical apparatus 1 according to another example of the offset arrangement. The medical apparatus 1 can furthermore comprise a distance varying element 26 extending between the first end 9 and the second end 10 of the positioning element 8 and fixable to the second end 10 for reducing or increasing the distance between said first end 9 and said second end 10 when the positioning element 8 passes from the first configuration to the second configuration and vice versa, the deformable element 11 surrounding (at least in part) the distance varying element 26.

In particular, as shown in figures 4A-4B, the distance varying element 26 can be a wire-shaped element extending longitudinally from the first end 9 to the second end 10 and being slidably movable inside at least the longitudinal sheath 5, for example passing through the central channel 14 of the first end 9.

The distance varying element 26 can be actuated when the deformable element 11 is in the extended configuration (figure 4A). In this configuration, the deformable threads 25 are symmetrically arranged around the treatment device 2 thereby forming a basket structure. The maximum width of this basket structure is denoted with the letter W in the figure. If the human cavity 4 inside which the medial apparatus 1 is inserted has the same or a smaller width, the deformable threads 25 would be in contact with the internal walls 3 of such a cavity 4. However, in case the cavity with is greater than the value of the maximum width W, the extended threads 25 would not be able to touch the internal walls 3 and the treatment device 2 would not be moved toward the central region of the cavity. To overcome this problem, the distance varying element 26 can be actuated. In this case, the distance between the first and second ends 9, 10 is reduced so that the deformable threads 15 can be further expanded due to compression and can get in close contact with the internal walls 3 of the body lumen being treated. As shown in figure 4B, the maximum width W is increased to a greater value denoted as W* in the figure.

Figures 5A-5G illustrate a possible approach to insert the medical apparatus 1 with the treatment device 2 in the human cavity 4. In this example, the cavity 4 is the duodenum area and the successive opening steps of the deformable element 11 are illustrated. Thanks to the operation of the medical apparatus 1, the treatment device 2, in this case an optical fiber laser, can be easily moved in the middle of the cavity 4. When the treatment device 2, i.e., the optical fiber laser, is well positioned in the center of the cavity 4, the laser can be activated in order to deliver the laser radiation to the internal surfaces 3 of the cavity 4. The central position of the treatment device 2 and the radial emission 27 guarantees the homogeneous distribution on the target surface. The quantity of energy applied depends on the power laser output, the geometry of the cavity and the desired effect on the target tissue.

According to figure 5A, a user, for example a surgeon, inserts the medical apparatus 1 combined with the treatment device 2 in the human cavity 4 and reaches the tract of the human cavity 4 needed to be treated. According to an example, the medical apparatus 1 has a longitudinal guiding axis GA. The guiding axis GA is an imaginary axis passing through the first and second guiding through-holes 12, 13 of the first and second ends 9, 10, respectively (as also shown in figure 1A). When the medical apparatus 1 is inserted in the cavity 4, the apparatus 1 is usually not centered in the cavity 4. As a matter of fact, the longitudinal guiding axis GA is shifted from a cavity central axis CA, in particular to the right according to the example of figure 5A. The central axis CA is an imaginary axis passing in the middle of the longitudinal cavity 4 and being almost equidistant from the internal walls 3 of said cavity 4. In figure 5B, the treatment device 2, i.e., the optical fiber laser, is extracted on the cavity 4. In this way, the tip 24 of the device 2 is moved away from the positioning element 8 and therefore from the longitudinal sheath 5. In figure 5C, the user can set the medical apparatus 1, and therefore the positioning element 8, in the second configuration. In this way, the deformable element 11 is expanded and the deformable threads 25 touch the internal walls 3 of the cavity 4, thereby moving, or rather pushing, the medical apparatus 1 with the treatment device 2 to the left towards the cavity central axis CA (see the horizontal arrow in the figure). In other words, according to an example, the passage from the first configuration to the second configuration causes a variation of the distance between the longitudinal guiding axis GA and the external walls 3 of the cavity 4. In figure 5D, the treatment device 2 is positioned in the middle of the cavity 4. As a matter of fact, the longitudinal guiding axis GA almost corresponds to the central axis CA. In figure 5E, the tip 24 of the treatment device 2 is further moved forward and is positioned on the correct longitudinal position following the user purpose. This procedure can be carried out using the camera 31 of the endoscopic device 7. In figure 5F, the treatment device 2, for example the laser system, is activated and the treatment device 2 delivers a laser radiation 27 by the radial/diffusive tip 27. In figure 5G, the treatment device 2 can be moved backwards simultaneously emitting the laser radiation 27 and delivering the energy on the cavity surface. In particular, according to an example, the treatment device 2 extends longitudinally and is configured to slide along the direction of the longitudinal sheath 5 through the first guiding through-hole 12 and the second guiding through-hole 13 when the apparatus 1 (e.g. the positioning element 8) is in the first configuration as well as in the second configuration.

The position in the middle of the cavity 4 of the treatment device 2 allows to have on the target tissue the same treatment, i.e., laser divergence and the same laser intensity causing the same desired effect. Without the correct position of the tip 24 the surfaces of the cavity 4 will be invested by different energy concentrations having nonhomogeneous ablation effects. Different laser intensities are at the origin of nonhomogeneous effects that could be introduce undesired effects. In this asymmetric configuration the laser effect could be too ablative on the closest areas to the tip 24 and too low effects on the areas far from the tip 24.

Figures 5A-5G illustrate the application of the apparatus 1 and in particular an auto-centering function of the apparatus 1 according to the offset arrangement. It is however noted that the same auto-centering function also applies to the apparatus 1 according to the coaxial arrangement. Accordingly, the steps described with reference to Fig. 5A-5G will not be repeated for the apparatus 1 according to the coaxial arrangement.

In applying such a technique with current flexible endoscopes, the medical apparatus 1 and treatment device 2 go through the same working channel 6, typically 3.7-4.2 mm diameter. The medical apparatus 1 and treatment device 2 inside to the external sheath 15, having a diameter of 2-3mm, are well supported on the working channel lumen 6. The working channel of a flexible endoscope is commonly centrally located along the endoscope body. A typical endoscope has its tip and the segment at the distal end of its shaft to be able to deflect in two opposite directions. The external sheath 15 with inside the medical apparatus 1 and treatment device 2 has a good flexibility without interference with the movements of the endoscopic device 7. When the external sheath 15 goes through the working channel 6 of the endoscopic device 7, the medical apparatus 1 and treatment device 2 do not interfere with each other when the surgeon manipulates them independent from each other. When the medical apparatus 1 and treatment device 2 go through the external sheath 15 these two instruments do not interfere with each other also thanks to the external jacket (typically Tetzel) of the surface of the optical fiber structure 33. Thus, the medical apparatus 1 and treatment device 2 can be manipulated free from mutual interference.

In examples, the ablating system 23 comprises a control unit for controlling the energy output of the treatment device 2 in case the treatment device 2 is a laser system. In addition or in alternative, the control unit serves for controlling the movement of the treatment device 2 through the first guiding through-hole 12 and the second guiding through-hole 13. In addition or in alternative, the control unit serves for controlling the movement of the treatment device 2 through the first guiding through-hole 12 and the second guiding through-hole 13 based on the energy output of the treatment device 2 in case the treatment device 2 is a laser system. Advantageously, to activate the control system a footswitch may be used by the user to start and stop the laser emission of a laser system connected to an optical fiber.

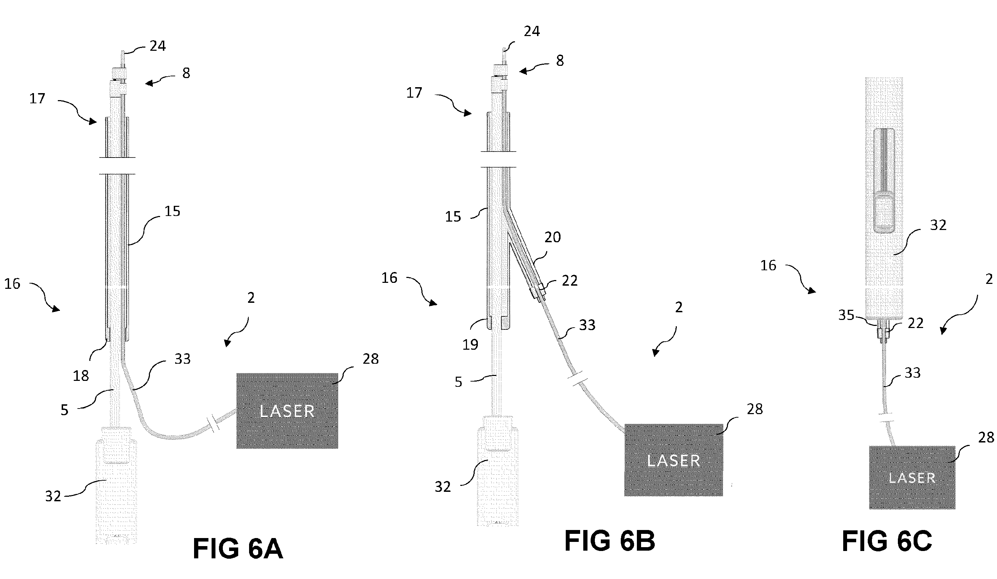

In an example, the external sheath 15 comprises a proximal inserting end 16 and a distal end 17. This is shown in figures 6A and 6B. Although these figures only illustrate the apparatus 1 according to the offset arrangement, the same characteristics also apply to the apparatus 1 according to the coaxial arrangement.

As shown in figure 6A, the proximal inserting end 16 can comprise a single port 18 for the passage of the longitudinal sheath 5 and of the treatment device 2. The treatment device 2 can comprise a laser generator 28 for the generation of the laser radiation passing through an optical fiber structure 33 coupled to the laser generator 28.

Alternatively, as shown in figure 6B, the proximal inserting end 16 can comprise a first port 19 for the passage of the longitudinal sheath 5 and a second port 20 for the passage of the treatment device 2. Also in this case, the treatment device 2 can comprise a laser generator 28 for the generation of the laser radiation passing through an optical fiber structure 33 coupled to the laser generator 28.

In both configurations of figures 6A and 6B, an end of the longitudinal sheath 5 is connected to an end piece element 32 that can be maneuvered by a user.

In addition, the proximal inserting end 16 can comprise at least a luer-lock element 22 for maintaining in position the element passing through the proximal inserting end 16.

For example, the luer-lock element 22 can fix the position of the optical fiber of the treatment device 2. During the insertion and retraction of the optical fiber, this element is unlocked.

Advantageously, the external sheath 15 can be made of a deformable material.

Figure 6C illustrates a connection configuration according to another example. Whereas figures 6A and 6B show a configuration wherein the ports 18, 19 and 20 are located at one end of the hand piece element 32, i.e. between the hand piece element 32 and the tip 24, figure 6C shows a configuration where a single port 35 is located on the opposite side of the hand piece element 32. The configurations of figures 6A-6B are suitable for an offset arrangement and the configuration of figure 6C is suitable for a coaxial arrangement.

Figures 7A and 7B illustrate the passage from the first configuration to the second configuration of the positioning element 8 according to an example of the offset arrangement. Th apparatus 1 further comprises an actuating element 21 for causing the passage from the first configuration to the second configuration and vice versa, wherein in particular one end of the deformable element 11 is connectable to said actuating element 21, the actuating element 21 being configured to slide along the direction of the longitudinal sheath 5. Using the hand piece element 32 by the movement of the actuating element or knob 21, the user can move the deformable threads 25 from the collapsed position (figure 7A) inside to the longitudinal sheath 5 to the open position (figure 7B) with the deformable threads 25 expanded outside to the sheath 5. Moving the knob 21 in the opposite side the user can compress the deformable threads 25 inside to the sheath 5. It is noted that the deformable threads 25 are connected to the actuating element 21. In other words, the deformable threads 25 connect the actuating element 21 to the second end 10 of the positioning element 8.

These figures describe a first distance d₁ and a second distance d₂, wherein the first distance d₁ represents the distance between the second end 10 and the longitudinal sheath 5, in particular an extremity of the longitudinal sheath 5 in the first (retracted) configuration, and the second distance d₂ represents the distance between the second end 10 and the longitudinal sheath 5, in particular an extremity of the longitudinal sheath 5 in the second (expanded) configuration. It is noted that the passage from the first configuration (Fig. 7A) to the second configuration (Fig. 7B) determines an increase of the distance between the second end 10 and the longitudinal sheath 5, i.e. d₁ < d₂. Figures 8A-8C illustrate the similar concept of figures 7A-7B according to another example, in particular according to the example wherein a distance varying element 26 is present. Differently from the configuration of figures 7A-7B, the actuating element comprises an upper actuating element 29 connected to the deformable threads 25 and a lower actuating element 30 connected to the distance varying element 26. Advantageously, the lower actuating element 30 allows the passage of the deformable threads 25, for example via a through-hole. Using the hand piece element 32 by the movement of both the upper and lower actuating elements 29, 30, the user can move the deformable threads 25 from the collapsed position (figure 8A) inside to the longitudinal sheath 5 to the open position (figure 8B) with the deformable threads 25 expanded outside to the sheath 5. Moving the lower actuating element 30 in the opposite side, the user can reduce the distance between the first end 9 and second end 10 of the positioning element 8, thereby increasing the width of the deformable element 11. Moving further moving the upper actuating element 29 backwards, the deformable threads 25 can be compressed inside to the sheath 5.

It is noted that the deformable threads 25 are connected to the upper actuating element 29 and the distance varying element 26 is connected to the lower actuating element 30. In other words, the deformable threads 25 connect the second end 10 of the positioning element 8 to the upper actuating element 29, whereas the distance varying element 26 connects the second end 10 of the positioning element 8 to the lower actuating element 30.

Also in this case, by passing from the first configuration (Fig. 8A) to the second configuration (Fig. 8C) the distance between the second end 10 and the longitudinal sheath 5 increases, i.e. d₁ < d₂.

In the coaxial arrangement, the distance varying element 26 can be a tube-shaped element extending longitudinally from the first end 9 to the second end 10 and being slidably movable inside at least the longitudinal sheath 5 and configured to receive the treatment device 2, the first guiding through-hole 12 and the second guiding through-hole 13 being part of the distance varying element 26. In other words, the first guiding through-hole 12 can correspond to the hole of the (tube-shaped) distance varying element 26 at the first end 9 of the positioning element 8. The second guiding through-hole 13 can correspond to the hole of the (tube-shaped) distance varying element 26 at the second end 10 of the positioning element 8. This is shown for example in figures 1D and 2C, in the coaxial arrangement, wherein the guiding axis GA coincides with the longitudinal axis LS of the longitudinal sheath 5.

Figures 9A-9C and figures 10A-10D illustrate the passage from the first (retracted) configuration to the second (expanded) configuration in the coaxial arrangement. It is noted that the passages described for the apparatus 1 according to the offset arrangement (figures 7A-B and 8A-C) also apply to the apparatus 1 according to the coaxial arrangement. Therefore, same steps and structural elements are not repeated here. As shown in figure 9A, in the first configuration the deformable element 11 has a retracted shape and extends longitudinally in the direction of the longitudinal sheath 5. In this case, the apparatus 1 can easily be inserted into the human cavity. By acting on the actuating element 21, the second end 10 is moved (pulled) towards the longitudinal sheath 5, i.e. the first end 9. Accordingly, the deformable elements 11 takes an expanded shape. It is noted that by passing from the first configuration (Fig. 9A) to the second configuration (Fig. 9C) the distance between the second end 10 and the longitudinal sheath 5 decreases, i.e. d₁ > d₂.

Figures 10A-10D describe the same procedure as in figures 9A-9C. The only difference here is that the apparatus 1 comprises an external sheath 15 surrounding the deformable element 11 for protection purposes (Fig. 10A). The deformable element 11 can be pushed out form the external sheath 15 at an external sheath extremity 34, wherein the deformable element 11 maintains its retracted shape (Fig. 10B). By acting on the actuating element 21, the second end 10 is moved (pulled) towards the longitudinal sheath 5, i.e. the first end 9. Accordingly, the deformable elements 11 takes an expanded shape. It is noted that by passing from the first configuration (Fig. 10B) to the second configuration (Fig. 10D) the distance between the second end 10 and the longitudinal sheath 5 decreases, i.e. d₁ > d₂ (not shown in the figure but similar to figures 9A-9C).

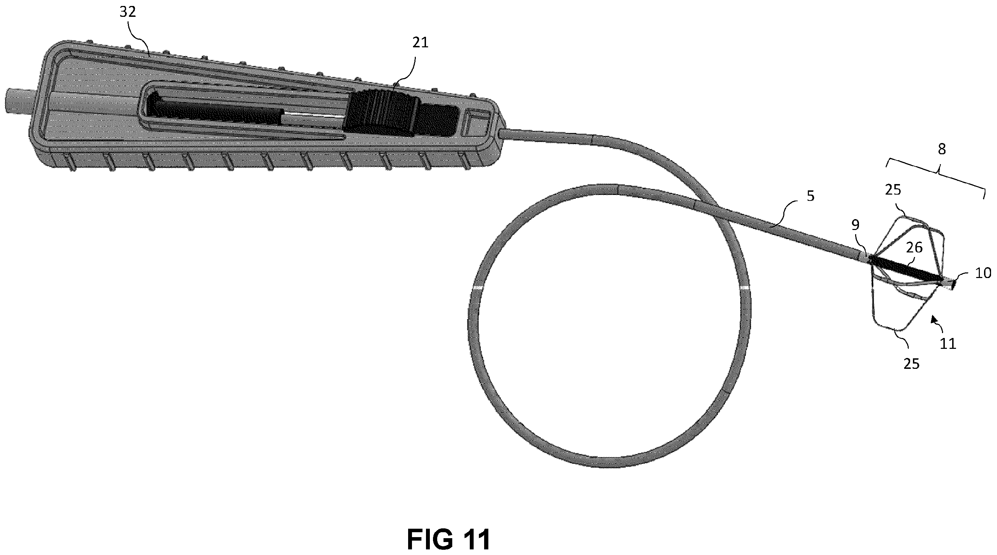

Figure 11 describes a possible example of an apparatus 1 according to the coaxial arrangement, wherein an end of the longitudinal sheath 5 is connected to an end piece element 32 that can be maneuvered by a user by the actuating element 21.

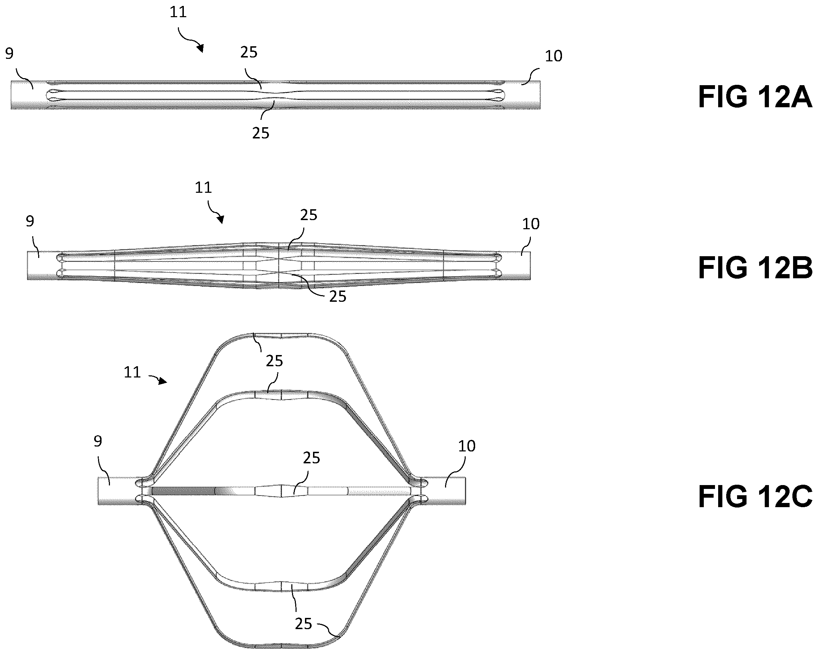

Figures 12A-C illustrate the shape variation of the deformable element 11 used in an apparatus 1 according to the coaxial configuration. In figure 12A, the deformable element 11 has an elongated shape, wherein the threads 25 are all parallel to each other without a specific shape. For example, the deformable element 11 can have this shape when located inside the external sheath 15 (Fig. 10A). In figure 12B, the deformable element 11 has always an elongated shape, but the threads 25 are slightly deformed (preshaped). For example, the deformable element 11 can have this shape when extracted from the external sheath 15 (Fig. 10B) or in the initial situation of figure 9A. In figure 12C, the deformable element 11 is expanded and can have the shape of a basket. For example, the deformable element 11 can have this shape in the second configuration (Fig. 9C, 10D). Between the pre-shape form and the expanded form, the deformable element 11 can assume intermediates shapes, such as for example in figures 8B, 9B, and 10C.

The apparatus 1 - both according to the offset arrangement and the coaxial arrangement
- can be used for a method for treating the internal walls of a longitudinal cavity, in particular a human cavity.

In particular, the method can be used for treating a human cavity in the gastro intestinal apparatus, with the purpose of targeting diseases of such apparatus, including Barrett's esophagus and other diseases and cancers formations. In addition or alternative, the method can be used for treating human cavities in the gastro intestinal apparatus, with the purpose of targeting metabolic diseases, in particular diabetes, nash, or fatty liver disease, and food intolerances.

The method can furthermore be used for treating a human cavity in the respiratory system, with the purpose of targeting respiratory diseases.

The method can also be used for treating a human cavity in the cardiac system, with the purpose of targeting cardiac diseases.

Although a variety of techniques and examples of such techniques have been described herein, these are provided by way of example only and many variations and modifications on such examples will be apparent to the skilled person and fall within the scope of the present invention, which is defined by the appended claims.

## Claims

1. Medical apparatus (1) for supporting and centering a treatment device (2) treating the internal walls (3) of a longitudinal cavity (4), in particular a human cavity, the apparatus (1) comprising:
a longitudinal sheath (5) insertable in a working channel (6) of an endoscopic device (7); and
a positioning element (8) comprising a first end (9) connectable to the longitudinal sheath (5) and having a central channel (14) as well as a first guiding through-hole (12), a second end (10) configured to be movable from the first end (9) and from the longitudinal sheath (5) and having a second guiding through-hole (13), and a deformable element (11) fixable to at least the second end (10);
wherein in a first configuration, or retracted configuration, the second end (10) of the positioning element (8) is at a first distance (d₁) from the longitudinal sheath (5), the first guiding through-hole (12) and the second guiding through-hole (13) being aligned for receiving the treatment device (2) and the deformable element (11) is completely compressed in the direction of the longitudinal sheath (5), and in a second configuration, or expanded configuration, the second end (10) of the positioning element (8) is spaced apart from the longitudinal sheath (5) at a second distance (d₂), wherein the first distance (d₁) is different from the second distance (d₂) and the deformable element (11) is radially expanded, thereby touching, at least partially, the internal walls (3) of the cavity (4) so that the first guiding through-hole (12) and the second guiding through-hole (13) are moved away from the internal walls (3) of the cavity (4) and the treatment device (2), when inserted into the first guiding through-hole (12) and the second guiding through-hole (13), is moved towards a central region of the cavity (4) and is maintained in said position during the treatment.

2. Apparatus (1) according to claim 1, wherein:
a. the first guiding through-hole (12) is coaxial with the central channel (14) and the second guiding through-hole (13) is centered on the second end (10); or
b. the central channel (14) is spaced from the first guiding through-hole (12) and the second guiding through-hole (13) is not centered on the second end (10).

3. . Apparatus (1) according to any one of the preceding claims, wherein:
a. the deformable element (11) is configured to slide through the central channel (14) of the first end (9) so that in the first configuration the second end (10) of the positioning element (8) is in contact with the first end (9) of the positioning element (8) so that the first distance (d₁) is smaller than the second distance (d₂) and the deformable element (11) is compressed inside the longitudinal sheath (5); or
b. the deformable element (11) is fixable also to the first end (9) of the positioning element (8) so that that in the first configuration the second end (10) of the positioning element (8) is moved away from the first end (9) of the positioning element (8) so that that the first distance (d₁) is greater than the second distance (d₂) and the deformable element (11) is compressed outside the longitudinal sheath (5).

4. . Apparatus according to any one of the preceding claims, further comprising a distance varying element (26) extending between the first end (9) and the second end (10) of the positioning element (8) and fixable to the second end (10) for reducing or increasing the distance between said first end (9) and said second end (10) when the positioning element (8) passes from the first configuration to the second configuration and vice versa, the deformable element (11) surrounding the distance varying element (26).

5. . Apparatus according to claim 4, wherein:
a. the distance varying element (26) is a tube-shaped element extending longitudinally from the first end (9) to the second end (10) and being slidably movable inside at least the longitudinal sheath (5) and configured to receive the treatment device (2), the first guiding through-hole 12 and the second guiding through-hole 13 being part of the distance varying element (26); or
b. the distance varying element (26) is a wire-shaped element extending longitudinally from the first end (9) to the second end (10) and being slidably movable inside at least the longitudinal sheath (5)

6. . Apparatus (1) according to any one of the proceeding claims, further comprising an external sheath (15) containing both the longitudinal sheath (5) and the treatment device (2), said external sheath (15) being insertable in the working channel (6) of the endoscopic device (7), the external sheath (15) comprising a proximal inserting end (16) and a distal end (17) and wherein:
a. the proximal inserting end (16) comprises a single port (18) for the passage of the longitudinal sheath (5) and of the treatment device (2); or
b. the proximal inserting end (16) comprises a first port (19) for the passage of the longitudinal sheath (5) and a second port (20) for the passage of the treatment device (2); and/or
c. the proximal inserting end (16) comprises at least a luer-lock element (22); and/or
d. the external sheath (15) is made of a deformable material.

7. Apparatus (1) according to any one of the preceding claims, wherein the deformable element (11) comprises a plurality of deformable threads (25), wherein:
a. the plurality of deformable threads (25) are made of a material having shape memory and superelasticity, in particular of a metal alloy of nickel and titanium; and/or
b. in the second configuration the deformable element (11) assumes a basket-shaped structure.

8. Apparatus (1) according to any one of the preceding claims, further comprising an actuating element (21) for causing the passage from the first configuration to the second configuration and vice versa, wherein in particular one end of the deformable element (11) is connectable to said actuating element (21), the actuating element (21) being configured to slide along the direction of the longitudinal sheath (5).

9. Apparatus (1) according to any one of the preceding claims, the apparatus having a longitudinal guiding axis (GA) passing through the first guiding through-hole 12 and the second guiding through-hole 13, wherein the passage from the first configuration to the second configuration causes a variation of the distance between said longitudinal guiding axis (GA) and the external walls (3) of the cavity (4).

10. Ablating and resurfacing system (23) for the ablation and the resurfacing of the superficial mucosae of a human cavity, in particular the gastro apparatus, more particularly the duodenum, the system comprising:
a medical apparatus (1) according to any one of claims 1 to 9, and
a treatment device (2) treating the internal walls (3) of the human cavity,
wherein in the first configuration of the apparatus (1), the treatment device (2) is extended through the first guiding through-hole (12) and the second guiding through-hole (13) and in the second configuration of the apparatus (1), the second end (10) of the positioning element (8) is moved along the treatment device (2) until the deformable element (11) is expanded and the treatment device (2) is moved away from the internal walls (3) of the cavity (4), in particular towards a central region of the cavity (4).

11. System (23) according to claim 10, wherein:
a. the treatment device (2) is a laser system, in particular emitting a radiation having a wavelength comprised between 1900 nm and 2100 nm or between 10200 nm and 10600nm; and/or
b. the treatment device (2) is an optical fiber laser system comprising a laser generator 28 coupled to an optical fiber structure 33 having a tip portion (24) for a diffusive or radial laser emission.

12. System (23) according to any one of claims 10 to 11, further comprising a control unit:
a. for controlling the energy output of the treatment device (2) in case the treatment device (2) is a laser system; and/or
b. for controlling the movement of the treatment device (2) through the first guiding through-hole (12) and the second guiding through-hole (13);and/or
c. for controlling the movement of the treatment device (2) through the first guiding through-hole (12) and the second guiding through-hole (13) based on the energy output of the treatment device (2) in case the treatment device (2) is a laser system.

13. System (23) according to any one of claims 10 to 12, wherein the treatment device (2) extends longitudinally and is configured to slide along the direction of the longitudinal sheath (5) through the first guiding through-hole (12) and the second guiding through-hole (13) when the apparatus (1) is in the first configuration as well as in the second configuration.

14. Endoscopic device (7) having at least a working channel (6) and comprising an apparatus (1) according to any one of claims 1 to 9 or a system (23) according to claims 10 to 13, the apparatus (1) or the system (23) being insertable in the working channel (6) of the endoscopic device (7).

## Patentansprüche

1. Medizinische Einrichtung (1) zum Unterstützen und Zentrieren einer Behandlungsvorrichtung (2) zum Behandeln der Innenwände (3) eines länglichen Hohlraums (4), insbesondere einer menschlichen Körperhöhle, wobei die Einrichtung (1) Folgendes umfasst:
eine längliche Hülle (5), die in einen Arbeitskanal (6) einer endoskopischen Vorrichtung (7) einführbar ist; und
ein Positionierungselement (8), das ein erstes Ende (9) umfasst, das mit der länglichen Höhle (5) verbindbar ist, und einen zentralen Kanal (14) sowie ein erstes Führungsdurchgangsloch (12) aufweist, ein zweites Ende (10), das dazu konfiguriert ist, von dem ersten Ende (9) und von der länglichen Hülle (5) aus bewegbar zu sein und ein zweites Führungsdurchgangsloch (13) aufweist, und ein verformbares Element (11), das mindestens an dem zweiten Ende (10) befestigbar ist;
wobei sich in einer ersten Konfiguration oder eingefahrenen Konfiguration das zweite Ende (10) des Positionierungselements (8) in einem ersten Abstand (d1) von der länglichen Hülle (5) befindet, wobei das erste Führungsdurchgangsloch (12) und das zweite Führungsdurchgangsloch (13) zu dem Aufnehmen der Behandlungsvorrichtung (2) ausgerichtet sind und das verformbare Element (11) in der Richtung der länglichen Hülle (5) vollständig komprimiert ist, und in einer zweiten Konfiguration oder ausgefahrenen Konfiguration, das zweite Ende (10) des Positionierungselements (8) in einem zweiten Abstand (d2) von der länglichen Hülle (5) beabstandet ist, wobei sich der erste Abstand (_{d1}) von dem zweiten Abstand (d₂) unterscheidet und das verformbare Element (11) radial ausgefahren ist und dadurch die Innenwände (3) des Hohlraums (4) mindestens teilweise derart berührt, dass das erste Führungsdurchgangsloch (12) und das zweite Führungsdurchgangsloch (13) von den Elementen (3) des Hohlraums (4) wegbewegt werden und die Behandlungsvorrichtung (2), wenn sie in das erste Führungsdurchgangsloch (12) und das zweite Führungsdurchgangsloch (13) eingeführt wird, in Richtung eines zentralen Bereichs des Hohlraums (4) wegbewegt wird und während der Behandlung in der Position gehalten wird.

2. Einrichtung (1) nach Anspruch 1, wobei:
a. das erste Führungsdurchgangsloch (12) zu dem zentralen Kanal (14) koaxial ist und das zweite Führungsdurchgangsloch (13) an dem zweiten Ende (10) zentriert ist; oder
b. der zentrale Kanal (14) von dem ersten Führungsdurchgangsloch (12) beabstandet ist, und das zweite Führungsdurchgangsloch (13) nicht auf dem zweiten Ende (10) zentriert ist.

3. Einrichtung (1) nach einem der vorstehenden Ansprüche, wobei:
a. das verformbare Element (11) dazu konfiguriert ist, durch den zentralen Kanal (14) des ersten Endes (9) derart zu gleiten, dass in der ersten Konfiguration das zweite Ende (10) des Positionierungselements (8) mit dem ersten Ende (9) des Positionierungselements (8) derart in Kontakt steht, dass der erste Abstand (d₁) kleiner als der zweite Abstand (d₂) ist und das verformbare Element (11) innerhalb der länglichen Hülle (5) komprimiert ist; oder
b. das verformbare Element (11) auch an dem ersten Ende (9) des Positionierungselements (8) derart befestigbar ist, dass in der ersten Konfiguration das zweite Ende (10) des Positionierungselements (8) von dem ersten Ende (9) des Positionierungselements (8) derart wegbewegt wird, dass der erste Abstand (d₁) größer als der zweite Abstand (d₂) ist und das verformbare Element (11) außerhalb der länglichen Hülle (5) komprimiert ist.

4. Einrichtung nach einem der vorstehenden Ansprüche, die weiter ein Abstandsvariationselement (26) umfasst, das sich zwischen dem ersten Ende (9) und dem zweiten Ende (10) des Positionierungselements (8) erstreckt und an dem zweiten Ende (10) befestigbar ist, um den Abstand zwischen dem ersten Ende (9) und dem zweiten Ende (10) zu verringern oder zu vergrößern, wenn das Positionierungselement (8) von der ersten Konfiguration in die zweite Konfiguration übergeht und umgekehrt, das verformbare Element (11) das Abstandsvariationselement (26) umschließt.

5. Einrichtung nach Anspruch 4, wobei:
a. das Abstandsvariationselement (26) ein rohrförmiges Element ist, das sich in Längsrichtung von dem ersten Ende (9) zu dem zweiten Ende (10) erstreckt und innerhalb mindestens der länglichen Hülle (5) verschiebbar ist und dazu konfiguriert ist, die Behandlungsvorrichtung (2) aufzunehmen, wobei das erste Führungsdurchgangsloch 12 und das zweite Führungsdurchgangsloch 13 Teil des Abstandsvariationselements (26) sind; oder
b. das Abstandsvariationselement (26) ein drahtförmiges Element, das sich in Längsrichtung von dem ersten Ende (9) zu dem zweiten Ende (10) erstreckt und innerhalb mindestens der länglichen Hülle (5) verschiebbar ist.

6. Einrichtung (1) nach einem der vorstehenden Ansprüche, die weiter eine äußere Hülle (15) umfasst, die sowohl die längliche Hülle (5) als auch die Behandlungsvorrichtung (2) enthält, wobei die äußere Hülle (15) in den Arbeitskanal (6) der endoskopischen Vorrichtung (7) einführbar ist und ein proximales Einführungsende (16) und ein distales Ende (17) umfasst, wobei:
a. das proximale Einführungsende (16) eine einzige Öffnung (18) für den Durchgang der länglichen Hülle (5) und der Behandlungsvorrichtung (2) umfasst; oder
b. das proximale Einführungsende (16) eine erste Öffnung (19) für den Durchgang der länglichen Hülle (5) und eine zweite Öffnung (20) für den Durchgang der Behandlungsvorrichtung (2) umfasst; und/oder
c. das proximale Einführungsende (16) mindestens ein Luer-Lock-Element (22) umfasst; und/oder
d. die äußere Hülle (15) aus einem verformbaren Material hergestellt ist.

7. Einrichtung (1) nach einem der vorstehenden Ansprüche, wobei das verformbare Element (11) eine Vielzahl von verformbaren Gewinden (25) umfasst, wobei:
a. die Vielzahl verformbarer Gewinde (25) aus einem Material hergestellt ist, das Formgedächtnis und Superelastizität aufweist, insbesondere aus einer Metalllegierung aus Nickel und Titan; und/oder
b. in der zweiten Konfiguration das verformbare Element (11) eine korbförmige Struktur annimmt.

8. Einrichtung (1) nach einem der vorstehenden Ansprüche, die weiter ein Betätigungselement (21) umfasst, um den Übergang von der ersten Konfiguration zu der zweiten Konfiguration und umgekehrt zu verursachen, wobei insbesondere ein Ende des verformbaren Elements (11) mit dem Betätigungselement (21) verbindbar ist, wobei das Betätigungselement (21) dazu konfiguriert ist, entlang der länglichen Hülle (5) zu gleiten.

9. Einrichtung (1) nach einem der vorstehenden Ansprüche, wobei die Einrichtung eine Längsführungsachse (GA) aufweist, die durch die erste Führungsdurchgangsbohrung 12 und die zweite Führungsdurchgangsbohrung 13 verläuft, wobei der Übergang von der ersten Konfiguration zu der zweiten Konfiguration eine Variation des Abstands zwischen der Längsführungsachse (GA) und den Außenwänden (3) des Hohlraums (4) bewirkt.

10. Ablations- und Oberflächenerneuerungssystem (23) zur Ablation und Oberflächenerneuerung der oberflächlichen Schleimhäute einer menschlichen Körperhöhle, insbesondere des Magen-Darm-Trakts, genauer genommen des Duodenums, wobei das System Folgendes umfasst:
eine medizinische Einrichtung (1) nach einem der Ansprüche 1 bis 9, und
eine Behandlungsvorrichtung (2), die die Innenwände (3) der menschlichen Körperhöhle behandelt,
wobei in der ersten Konfiguration der Einrichtung (1) die Behandlungsvorrichtung (2) durch die erste Führungsdurchgangsloch (12) und die zweite Führungsdurchgangsloch (13) verlängert ist, und in der zweiten Konfiguration der Einrichtung (1) das zweite Ende (10) des Positionierungselements (8) entlang der Behandlungsvorrichtung (2) bewegt wird, bis das verformbare Element (11) aufgeweitet ist und die Behandlungsvorrichtung (2) von den Innenwänden (3) des Hohlraums (4) wegbewegt wird, insbesondere in Richtung eines zentralen Bereichs des Hohlraums (4).

11. System (23) nach Anspruch 10, wobei:
a. die Behandlungsvorrichtung (2) ein Lasersystem ist, das insbesondere eine Strahlung emittiert, die eine Wellenlänge zwischen 1900 nm und 2100 nm oder zwischen 10200 nm und 10600 nm aufweist; und/oder
b. die Behandlungsvorrichtung (2) ein optisches Faserlasersystem ist, das einen Lasergenerator 28 umfasst, der mit einer optischen Faserstruktur 33 gekoppelt ist, die einen Spitzenabschnitt (24) für eine diffuse oder radiale Laseremission aufweist.

12. System (23) nach einem der Ansprüche 10 oder 11, das weiter eine Steuereinheit umfasst:
a. zum Steuern der Energieabgabe der Behandlungsvorrichtung (2), falls es sich bei der Behandlungsvorrichtung (2) um ein Lasersystem handelt; und/oder
b. zum Steuern der Bewegung der Behandlungsvorrichtung (2) durch das erste Führungsdurchgangsloch (12) und das zweite Führungsdurchgangsloch (13); und/oder
c. zum Steuern der Bewegung der Behandlungsvorrichtung (2) durch das erste Führungsdurchgangsloch (12) und das zweite Führungsdurchgangsloch (13) basierend auf der Energieausgabe der Behandlungsvorrichtung (2), falls es sich bei der Behandlungsvorrichtung (2) um ein Lasersystem handelt.

13. System (23) nach einem der Ansprüche 10 bis 12, wobei sich die Behandlungsvorrichtung (2) in Längsrichtung erstreckt und dazu konfiguriert ist, entlang der Richtung der länglichen Hülle (5) durch das erste Führungsdurchgangsloch (12) und das zweite Führungsdurchgangsloch (13) zu gleiten, wenn sich die Einrichtung (1) in der ersten Konfiguration sowie in der zweiten Konfiguration befindet.

14. Endoskopische Vorrichtung (7), die mindestens einen Arbeitskanal (6) aufweist und eine Einrichtung (1) nach einem der Ansprüche 1 bis 9 oder ein System (23) nach den Ansprüchen 10 bis 13 umfasst, wobei die Einrichtung (1) oder das System (23) in den Arbeitskanal (6) der endoskopischen Vorrichtung (7) einführbar ist.

## Revendications

1. Appareil médical (1) destiné à supporter et à centrer un dispositif de traitement (2) traitant les parois internes (3) d'une cavité longitudinale (4), notamment une cavité humaine, l'appareil (1) comprenant :
une gaine longitudinale (5) insérable dans un canal de travail (6) d'un dispositif endoscopique (7) ; et
un élément de positionnement (8) comprenant une première extrémité (9) pouvant être reliée à la gaine longitudinale (5) et présentant un canal central (14) ainsi qu'un premier trou de guidage traversant (12), une seconde extrémité (10) configurée pour être mobile à partir de la première extrémité (9) et de la gaine longitudinale (5) et présentant un second trou de guidage traversant (13), et un élément déformable (11) pouvant être fixé au moins à la seconde extrémité (10) ;
dans lequel, dans une première configuration, ou configuration rétractée, la seconde extrémité (10) de l'élément de positionnement (8) se trouve à une première distance (d₁) de la gaine longitudinale (5), le premier trou de guidage traversant (12) et le second trou de guidage traversant (13) étant alignés pour recevoir le dispositif de traitement (2), et l'élément déformable (11) est complètement comprimé dans le sens de la gaine longitudinale (5) et, dans une seconde configuration, ou configuration déployée, la seconde extrémité (10) de l'élément de positionnement (8) est espacée de la gaine longitudinale (5) d'une seconde distance (d₂), dans lequel la première distance (d₁) est différente de la seconde distance (d₂), et l'élément déformable (11) est déployé radialement, touchant ainsi, au moins partiellement, les parois internes (3) de la cavité (4) de sorte que le premier trou de guidage traversant (12) et le second trou de guidage traversant (13) s'éloignent des parois internes (3) de la cavité (4) et le dispositif de traitement (2), lors de son insertion dans le premier trou de guidage traversant (12) et le second trou de guidage traversant (13), se rapproche d'une région centrale de la cavité (4) et est maintenu dans cette position pendant le traitement.

2. Appareil (1) selon la revendication 1, dans lequel :
a. le premier trou de guidage traversant (12) est coaxial avec le canal central (14) et le second trou de guidage traversant (13) est centré sur la seconde extrémité (10) ; ou
b. le canal central (14) est espacé du premier trou de guidage traversant (12) et le second trou de guidage traversant (13) n'est pas centré sur la seconde extrémité (10).

3. Appareil (1) selon l'une quelconque des revendications précédentes, dans lequel :
a. l'élément déformable (11) est configuré pour glisser à travers le canal central (14) de la première extrémité (9) de sorte que, dans la première configuration, la seconde extrémité (10) de l'élément de positionnement (8) soit en contact avec la première extrémité (9) de l'élément de positionnement (8) afin que la première distance (d₁) soit inférieure à la seconde distance (d₂) et que l'élément déformable (11) soit comprimé à l'intérieur de la gaine longitudinale (5) ; ou
b. l'élément déformable (11) peut également être fixé à la première extrémité (9) de l'élément de positionnement (8) de sorte que, dans la première configuration, la seconde extrémité (10) de l'élément de positionnement (8) s'éloigne de la première extrémité (9) de l'élément de positionnement (8) afin que la première distance (d₁) soit supérieure à la seconde distance (d₂) et que l'élément déformable (11) soit comprimé à l'extérieur de la gaine longitudinale (5).

4. Appareil selon l'une quelconque des revendications précédentes, comprenant en outre un élément de variation de distance (26) s'étendant entre la première extrémité (9) et la seconde extrémité (10) de l'élément de positionnement (8) et pouvant être fixé à la seconde extrémité (10) pour réduire ou augmenter la distance entre ladite première extrémité (9) et ladite seconde extrémité (10) lorsque l'élément de positionnement (8) passe de la première configuration à la seconde configuration et vice versa, l'élément déformable (11) entourant l'élément de variation de distance (26).

5. Appareil selon la revendication 4, dans lequel :
a. l'élément de variation de distance (26) est un élément tubulaire s'étendant longitudinalement de la première extrémité (9) à la seconde extrémité (10) et pouvant coulisser à l'intérieur au moins de la gaine longitudinale (5) et configuré pour recevoir le dispositif de traitement (2), le premier trou de guidage traversant 12 et le second trou de guidage traversant 13 faisant partie de l'élément de variation de distance (26) ; ou
b. l'élément de variation de distance (26) est un élément en forme de fil s'étendant longitudinalement de la première extrémité (9) à la seconde extrémité (10) et pouvant coulisser à l'intérieur au moins de la gaine longitudinale (5)

6. Appareil (1) selon l'une quelconque des revendications précédentes, comprenant en outre une gaine externe (15) contenant à la fois la gaine longitudinale (5) et le dispositif de traitement (2), ladite gaine externe (15) pouvant être insérée dans le canal de travail (6) du dispositif endoscopique (7), la gaine externe (15) comprenant une extrémité d'insertion proximale (16) et une extrémité distale (17), et dans lequel :
a. l'extrémité d'insertion proximale (16) comprend un seul orifice (18) pour le passage de la gaine longitudinale (5) et du dispositif de traitement (2) ; ou
b. l'extrémité d'insertion proximale (16) comprend un premier orifice (19) pour le passage de la gaine longitudinale (5) et un second orifice (20) pour le passage du dispositif de traitement (2) ; et/ou
c. l'extrémité d'insertion proximale (16) comprend au moins un élément luer-lock (22) ; et/ou
d. la gaine externe (15) est constituée d'un matériau déformable.

7. Appareil (1) selon l'une quelconque des revendications précédentes, dans lequel l'élément déformable (11) comprend une pluralité de fils déformables (25), dans lequel :
a. la pluralité de fils déformables (25) sont constitués d'un matériau présentant une mémoire de forme et une superélasticité, en particulier d'un alliage métallique de nickel et de titane ; et/ou
b. dans la seconde configuration, l'élément déformable (11) prend une structure en forme de panier.

8. Appareil (1) selon l'une quelconque des revendications précédentes, comprenant en outre un élément d'actionnement (21) pour provoquer le passage de la première configuration à la seconde configuration et vice versa, dans lequel en particulier une extrémité de l'élément déformable (11) peut être reliée audit élément d'actionnement (21), l'élément d'actionnement (21) étant configuré pour glisser dans le sens de la gaine longitudinale (5).

9. Appareil (1) selon l'une quelconque des revendications précédentes, l'appareil présentant un axe de guidage longitudinal (GA) passant par le premier trou de guidage traversant 12 et le second trou de guidage traversant 13, dans lequel le passage de la première configuration à la seconde configuration provoque une variation de la distance entre ledit axe de guidage longitudinal (GA) et les parois externes (3) de la cavité (4).

10. Système d'ablation et de resurfaçage (23) pour l'ablation et le resurfaçage des muqueuses superficielles d'une cavité humaine, en particulier de l'appareil gastrointestinal, plus particulièrement du duodénum, le système comprenant :
un appareil médical (1) selon l'une quelconque des revendications 1 à 9, et
un dispositif de traitement (2) traitant les parois internes (3) de la cavité humaine,
dans lequel, dans la première configuration de l'appareil (1), le dispositif de traitement (2) est étendu à travers le premier trou de guidage traversant (12) et le second trou de guidage traversant (13) et, dans la seconde configuration de l'appareil (1), la seconde extrémité (10) de l'élément de positionnement (8) est déplacée le long du dispositif de traitement (2) jusqu'à ce que l'élément déformable (11) soit déployé et que le dispositif de traitement (2) s'éloigne des parois internes (3) de la cavité (4), en particulier vers une région centrale de la cavité (4).

11. Système (23) selon la revendication 10, dans lequel :
a. le dispositif de traitement (2) est un système laser, émettant en particulier un rayonnement présentant une longueur d'onde comprise entre 1900 nm et 2100 nm ou entre 10 200 nm et 10 600 nm ; et/ou
b. le dispositif de traitement (2) est un système laser à fibre optique comprenant un générateur laser 28 couplé à une structure de fibre optique 33 présentant une partie d'extrémité (24) pour une émission laser diffuse ou radiale.

12. Système (23) selon l'une quelconque des revendications 10 à 11, comprenant en outre une unité de commande :
a. pour commander la sortie d'énergie du dispositif de traitement (2) dans le cas où le dispositif de traitement (2) est un système laser ; et/ou
b. pour commander le déplacement du dispositif de traitement (2) à travers le premier trou de guidage traversant (12) et le second trou de guidage traversant (13) ; et/ou
c. pour commander le déplacement du dispositif de traitement (2) à travers le premier trou de guidage traversant (12) et le second trou de guidage traversant (13) sur la base de la sortie d'énergie du dispositif de traitement (2) dans le cas où le dispositif de traitement (2) est un système laser.

13. Système (23) selon l'une quelconque des revendications 10 à 12, dans lequel le dispositif de traitement (2) s'étend longitudinalement et est configuré pour glisser dans le sens de la gaine longitudinale (5) à travers le premier trou de guidage traversant (12) et le second trou de guidage traversant (13) lorsque l'appareil (1) est dans la première configuration ainsi que dans la seconde configuration.

14. Dispositif endoscopique (7) présentant au moins un canal de travail (6) et comprenant un appareil (1) selon l'une quelconque des revendications 1 à 9 ou un système (23) selon les revendications 10 à 13, l'appareil (1) ou le système (23) pouvant être inséré dans le canal de travail (6) du dispositif endoscopique (7).
